# EUROPÄISCHE PATENTSCHRIFT

(11) **EP 0 680 325 B1**
(45) Veröffentlichungstag und Bekanntmachung des Hinweises auf die Patenterteilung: **17.04.2002**
(21) Anmeldenummer: 94905024.9
(22) Anmeldetag: 10.01.1994
(51) Int. Cl.: A61K 31/55, A61K 9/70

(54) **TRANSDERMALES THERAPEUTISCHES SYSTEM MIT GALANTHAMIN ALS WIRKSAMEN BESTANDTEIL**
TRANSDERMAL THERAPEUTIC SYSTEM WITH GALANTHAMINE AS ACTIVE INGREDIENT
SYSTEME THERAPEUTIQUE TRANSDERMIQUE AVEC DE LA GALANTHAMINE COMME COMPOSANT ACTIF

(30) Priorität: 23.01.1993 DE 4301783
(43) Veröffentlichungstag der Anmeldung: 08.11.1995
(73) Patentinhaber: LTS LOHMANN Therapie-Systeme AG, 56626 Andernach (DE)
(72) Erfinder: HILLE, Thomas, D-56564 Neuwied (DE); DEURER, Lothar, D-56077 Koblenz (DE)
(74) Vertreter: Flaccus, Rolf-Dieter, Dr.
(86) Internationale Anmeldenummer: EP9400054
(87) Internationale Veröffentlichungsnummer: WO9416707

(56) Entgegenhaltungen:
- EP-A- 0 449 247
- EP-A- 0 515 301

## Beschreibung

Die Erfindung betrifft ein transdermales therapeutisches System (TTS), das als aktiven Bestandteil Galanthamin (4 a, 5, 9, 10, 11, 12-Hexahydro-3-methoxy-11-methyl-6 H-benzofuro (3 a, 3, 2-ef) (2) benazepin -6-ol) enthält.

Aufgrund seiner pharmakologischen Eigenschaften gehört Galanthamin zur Gruppe der reversibel wirkenden Cholinesterasehemmstoffen. Es steht in seinen Wirkungen dem Physostigmin und dem Neostigmin nahe, besitzt jedoch auch spezifische Eigenschaften. Galanthamin hat eine 3- bis 6mal größere therapeutische Breite, weil es nicht so toxisch wie Physostigmin oder Neostigmin ist.

Dieser Vorteil wiegt seine dosisbezogenen etwas geringere Cholinesterasehemmwirkung auf.

Hauptanwendungsgebiete des Galanthamins sind die Behandlung des Engwinkelglaukoms und der Einsatz als Antidot nach Curare-Applikationen. Versuchsweise wird Galanthamin bei der Alzheimerschen Krankheit eingesetzt.

In jüngster Zeit wurde Galanthamin zur Behandlung der Alkoholabhängigkeit eingesetzt (Opitz, K. DE 40 10 079).

Sowohl die Therapie der Alzheimerschen Krankheit als auch der Alkoholabhängigkeit erfordern langwirksame und den besonderen Umständen beider Krankheiten Rechnung tragende Arzneiformen. Schwierige Therapieschemata oder Dauerinfusionen kommen aus naheliegenden Gründen nicht in Frage.

Vielmehr ist ein TTS die Arzneiform der Wahl; dennoch ist es bis heute nicht gelungen, Galanthamin transdermal in der erforderlichen Menge zur Resorption zu bringen.

EP 0 449 247 A2 offenbart die Verwendung von Galanthamin zur Behandlung des Alkoholismus, wobei u.a. die transdermale Applikation vorgeschlagen wird. Die erfindungsgemäße, spezifische zusammensetzung des TTS ist nicht offenbart.

EP 0 144 486 A2 beschreibt allgemein transdermale Systeme, u. a. bestehend auf Basis von Polyacrylaten. Galanthamin ist als Wirkstoff nicht offenbart.

Aufgabe der Erfindung ist daher die Bereitstellung von Galanthamin oder eines seiner pharmazeutisch verträglichen Salze in Form eines transdermalen therapeutischen Systems, das Galanthamin oder dessen pharmazeutisch verträgliches Salz über einen Zeitraum von mindestens 24 Stunden kontrolliert abgibt und gewährleistet, daß das Galanthamin sich während der Lagerung des vorgefertigten transdermalen therapeutischen Systems nicht merklich zersetzt und sicherstellt, daß das Galanthamin im geforderten Ausmaß in vivo durch menschliche Haut penetriert.

Diese Aufgabe wird mit der Erfindung in überraschender Weise gelöst durch ein transdermales therapeutisches System nach Anspruch 1.

Diese Lösung ist umso erstaunlicher, als Galanthamin strukturell den Opiaten sehr ähnlich ist. Opiate gelten als Substanzklasse, die die menschliche Haut nur unzureichend zu durchdringen vermögen.

Ohne den Rahmen der Erfindung einzuschränken, sollen unter pharmazeutisch verträglichen Salzen des Galanthamins vorzugsweise dessen Hydrobromid und Hydrochlorid verstanden werden.
Vorteilhafte Ausgestaltungen der Erfindung sind entsprechend den Merkmalen der Unteransprüche vorgesehen.

Die wirkstoffundurchlässige Rückschicht kann aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden.

Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einer mit Aluminium bedampften Folie.

Die Reservoirschicht besteht aus einer Polymermatrix und dem Wirkstoff, wobei die Polymermatrix den Zusammenhalt des Systems gewährleistet. Sie besteht aus einem Grundpolymer und gegebenenfalls den üblichen Zusätzen. Die Auswahl des Grundpolymers richtet sich nach den chemischen und physikalischen Eigenschaften des Galanthamins. Beispielhafte Polymere sind Kautschuk, kautschukähnliche, synthetische Homo-, Co- oder Blockpolymere, Polyacrylsäureester und deren Copolymere, Polyurethane und Silikone. Grundsätzlich kommen alle Polymere in Frage, die bei der Herstellung von Haftklebern eingesetzt werden können und physiologisch unbedenklich sind. Besonders bevorzugt sind solche, die aus Blockcopolymeren auf Basis von Styrol und 1,3-Dienen, Polyisobutylenen, Silikonen, Polymeren auf Acrylat- und/oder Methacrylat bestehen.

Von den Blockcopolymeren auf Basis von Styrol und 1,3-Dienen werden ganz besonders linare Styrol-Isopren- oder Styrol-Butadien-Blockcopolymere eingesetzt.

Als Polymere auf Acrylat-Basis werden selbstvernetzende Acrylatcopolymere aus 2-Ethylhexylacrylat, Vinylacetat und Acrylsäure mit bzw. nicht selbstvernetzende Acrylatcopolymere ohne Titanchelatester bevorzugt.

Als Polymere, die dem Grundpolymer zugesetzt werden, kommen Polymethacrylate und Polyvinyle in Frage.

Als Methacrylate werden Copolymere auf Basis von Dimethylaminoethylmethacrylaten und neutralen Methacrylsäureestern bevorzugt.
Als Polyvinyle werden vorzugsweise Polyvinylpyrrolidone und Polyvinylalkohole eingesetzt.

Die Wahl des Weichmachers richtet sich nach dem Polymer. Besonders geeignet sind höhere Alkohole wie Dodecanol, Undecanol, Octanol, Oleylalkohol und 2-Octyldodecanol, Ester von Carbonsäuren, wobei die Alkoholkomponente auch ein polyethoxylierter Alkohol sein kann, Diester von Dicarbonsäuren, z.B. Di-n-butyladipat sowie Triglyceride, insbesondere mittelkettige Triglyceride der Capryl/Caprinsäuren des Kokosöls. Weitere Beispiele für einen geeigneten Weichmacher sind mehrwertige Alkohole, z.B. Glycerin und Propandiol-(1, 2) u.a., die auch durch Polyethylenglykole verethert sein können.

Als Penetrationsförderer kommen alle Carbonsäuren in Frage, die physiologisch unbedenklich sind. Besonders geeignet sind Octansäure, Lävulinsäure, Undecensäure, Ölsäure sowie Stearinsäure und ihre Isomeren.

Die Art der üblichen Zusätze hängt vom eingesetzten Polymer ab: Nach ihrer Funktion lassen sie sich einteilen in beispielsweise Klebrigmacher, Stabilisatoren, Trägerstoffe und Füllstoffe. Die hierfür in Frage kommenden physiologisch unbedenklichen Substanzen sind dem Fachmann bekannt.

Die Reservoirschicht besitzt eine solche Eigenklebrigkeit, daß ein dauernder Kontakt zur Haut sichergestellt ist.

Die ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, daß sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Das erfindungsgemäße transdermale therapeutische System wird hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das Lösemittel oder die Lösemittel entfernt wird/werden. Anschließend wird die Klebeschicht mit einer entsprechenden Schutzschicht versehen.

Auch der umgekehrte Weg, daß die Kleberlösung auf die Schutzschicht aufgestrichen wird, ist grundsätzlich möglich. Man entfernt auch in diesem Fall die Lösungsmittel und deckt dann anschließend mit der Rückschicht ab.

Die Erfindung wird durch die folgenden Beispiele erläutert:

### Beispiel 1

Je 10,0 g Octansäure und 10,0 g Isopropylmyristat werden unter Rühren gemischt. Anschließend werden 10,0 g Galanthamin eingetragen; man rührt bis zum vollständigen Auflösen des Feststoffs (ca. 30 min., visuelle Kontrolle). Danach werden unter Rühren 130,0 g eines selbstvernetzenden Acrylatcopolymeren aus 2-Ethylhexylacrylat, Vinylacetat und Acrylsäure 46%ig in einem Lösungsmittelgemisch (Ethylacetat: Heptan: Isopropanol: Toluol: Acetylaceton 37 : 26 : 26 : 4 : 1) zugegeben; es wird homogenisiert. Danach werden unter Rühren noch zusätzlich 10 g eines Methacrylatcopolymers auf Basis von Dimethylaminomethacrylat und neutralen Methacrylsäureestern eingestreut und 3 Stunden lang bei Raumtemperatur gerührt. Der Verdunstungsverlust wird ausgeglichen.

Es resultieren 150 g 52,8%ige (G/G) wirkstoffhaltige Kleberlösung, die mit einem 350 µm Rakel auf eine aluminisierte und silikonisierte Polyethylenfolie gestrichen wird. Nachdem die Lösungsmittel durch 30minütiges Trocknen bis 60°C entfernt wurden, deckt man den Kleberfilm mit einer Polyesterfolie 15 µm ab. Mit geeigneten Schneidewerkzeugen stanzt man eine Fläche von 16 cm² aus und entfernt die Ränder durch Abgittern. Die Freisetzung dieses und der anderen Rezepturbeispiele sind in der Tabelle angegeben; dort sind sowohl die kontrollierte Freisetzung in eine physiologische Kochsalzlösung als auch durch exzidierte Nagetierhaut aufgeführt.

Alle weiteren Beispiele werden nach dem unter Beispiel 1 angegebenen Schema angefertigt. Zunächst werden immer die flüssigen Bestandteile gemischt, dann die Galanthaminbase eingestreut. Nach deren Auflösung wird die Kleberlösung zugefügt. In der folgendenden Tabelle sind die Rezepturbestandteile nach Trocknen aufgeführt.

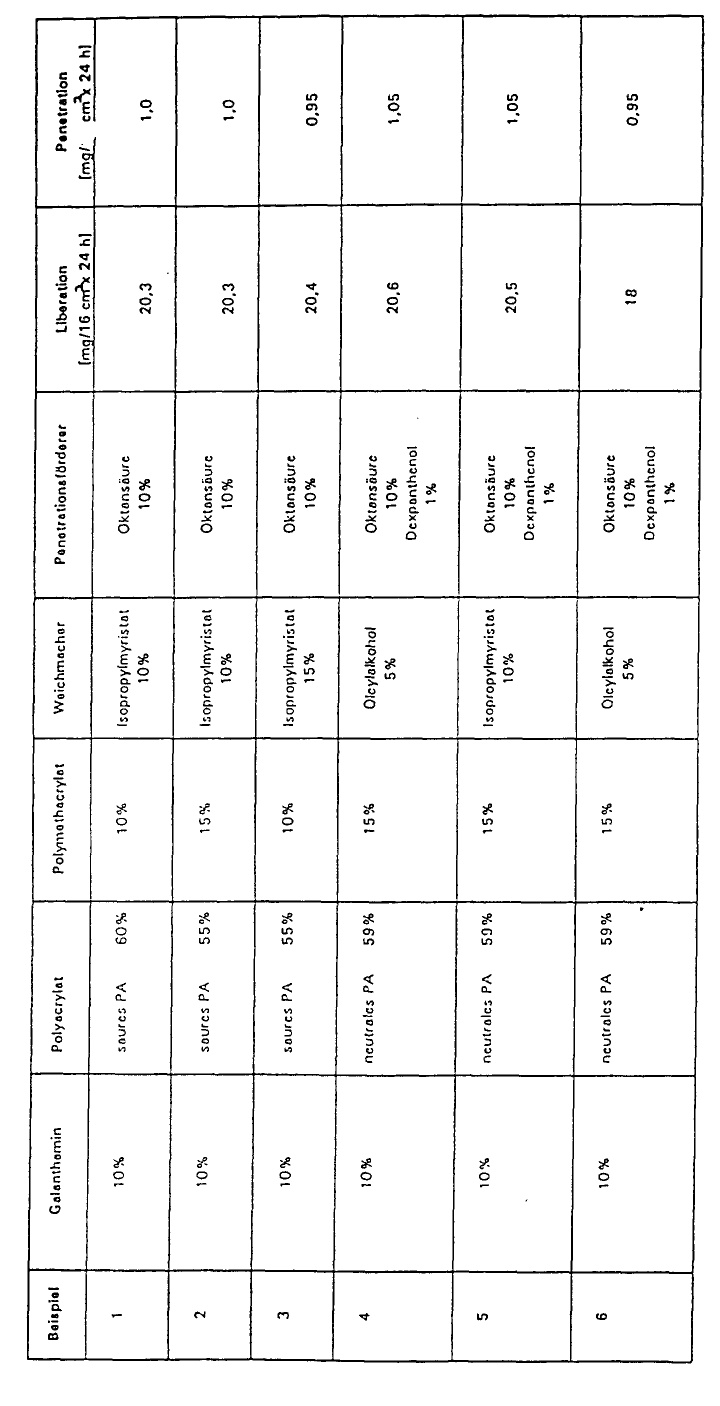

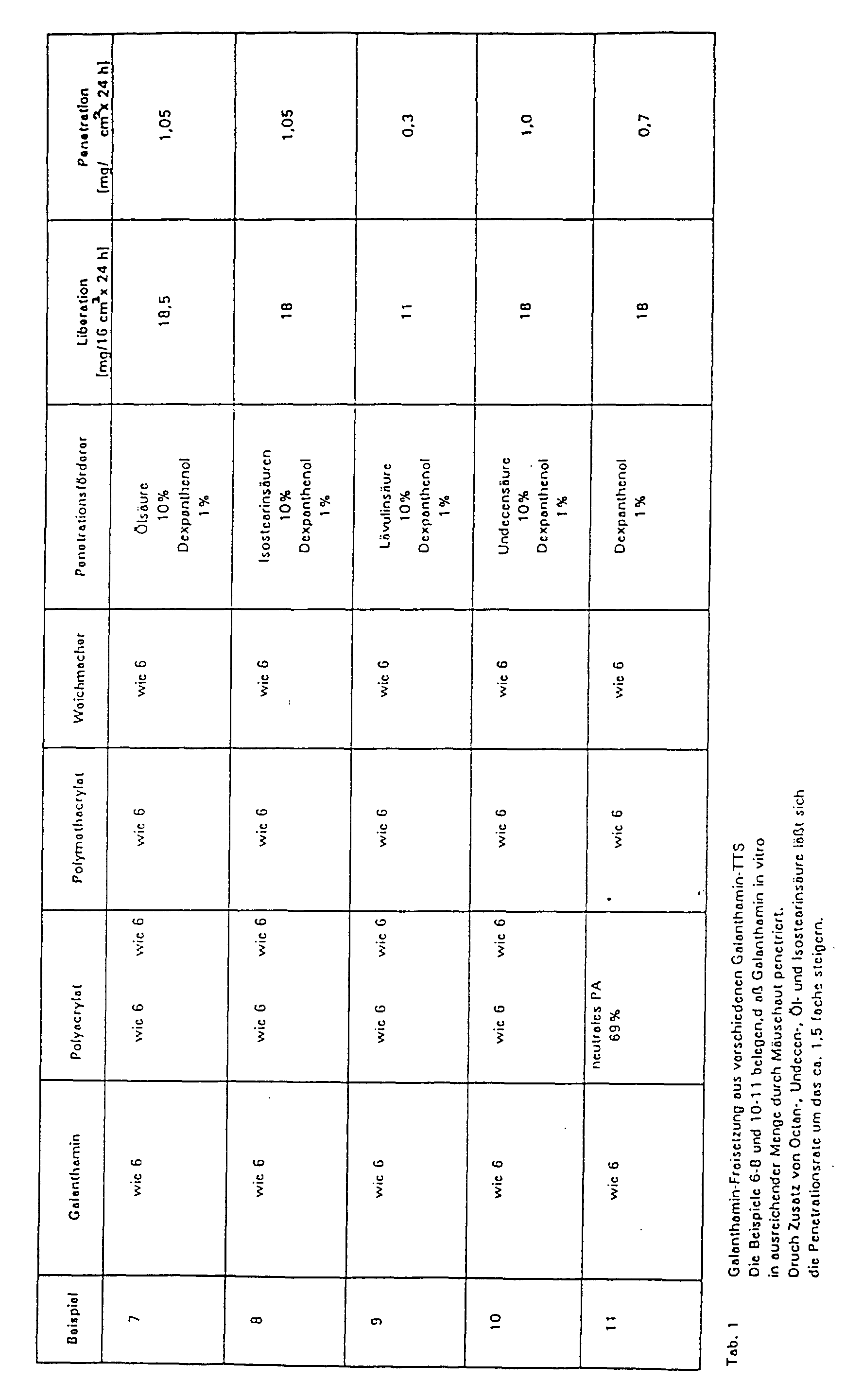

Die in vitro-Freisetzung wurde in einem Schüttelwasserbad bei 37°C bestimmt. Das Akzeptormedium waren 100 ml physiologische Kochsalzlösung, die nach 2, 4 und 8 Stunden komplett ausgewechselt wurden. Die Konzentration wurde nach 2, 4 und 8 und 24 Stunden per HPLC bestimmt. Die Penetration an der Mäusehaut wurde an Franz'schen Diffusionszellen gemessen.

In der Tabelle bedeuten:

| | |
|---|---|
| saures Polyacrylat (PA) | Acrylatcopolymeres aus 2-Ethylhexylacrylat, Vinylacetat und Acrylsäure mit Säurezahl 40 |
| neutrales PA | Acrylatcopolymeres aus 2-Ethylhexylacrylat, Vinylacetat und Acrylsäure mit Säurezahl um 1. |
| Polymethacrylat | Copolymeres mit basischem Charakter auf Basis von Dimethylaminomethacrylat und neutralen Methacrylsäureestern (KOH-Zahl 180). |

## Patentansprüche

1. Transdermales therapeutisches System (TTS) zur Verabreichung von Galanthamin an die Haut mit einer wirkstoffundurchlässigen Rückschicht und einer haftklebenden Reservoirschicht, **dadurch gekennzeichnet, daß** die Reservoirschicht 40-80 Gew.-% Polymermaterial aüf Acrylatbasis, 0,1-30 Gew.-% Weichmacher aus der Gruppe der Alkohole, Ester und (poly)ethoxylierten Verbindungen und 0,1-30 Gew.-% Galanthaminbase oder eines seiner pharmazeutisch akzeptablen Salze umfaßt.

2. TTS nach Anspruch 1, **dadurch gekennzeichnet, daß** die haftklebende Reservoirschicht weiters 0,1-30 Gew.-% Penetrationsförderer enthält.

3. TTS nach Anspruch 2, **dadurch gekennzeichnet, daß** der Penetrationsförderer eine Carbonsäure ist.

4. TTS nach Anspruch 1, **dadurch gekennzeichnet, daß** das Polymermaterial auf Acrylatbasis ein Polymerisationsprodukt aus Acrylsäure und ihren Estern oder Methacrylsäure und ihren Estern ist.

5. TTS nach Anspruch 4, **dadurch gekennzeichnet daß** die Ester der Acrylsäure als alkoholische Bestandteile geradkettige oder verzweigte Alkohole mit 4-10 Kohlenstoffen umfassen.

6. TTS nach Anspruch 4, **dadurch gekennzeichnet, daß** die Ester der Acrylsäure als alkoholische Bestandteile Alkohole mit 2-4 Kohlenstoffen umfassen.

7. TTS nach Anspruch 4, **dadurch gekennzeichnet, daß** die Ester der Methacrylsäure als alkoholischen Anteil Aminoalkohole aufweisen.

8. TTS nach Anspruch 4, **dadurch gekennzeichnet, daß** das Polymermaterial auf Acrylatbasis ein ggf. selbstvernetzendes Copolymeres auf Acrylatbasis ist.

9. TTS nach Anspruch 1, **dadurch gekennzeichnet, daß** das System mit einer wiederablösbaren Schutzschicht versehen ist.

## Claims

1. Transdermal therapeutic system (TTS) for the administration of galanthamine to the skin having a backing layer which is impermeable to active substances and a pressure-sensitive adhesive reservoir layer, **characterized in that** the reservoir layer comprises 40-80%-wt. acrylate-based polymer material, 0.1 - 30%-wt. plasticizer, and 0.1 - 30%-wt. galanthamine base or one of the pharmaceutically acceptable salts thereof.

2. The TTS according to claim 1, **characterized in that** the pressure-sensitive adhesive reservoir layer further comprises 0.1 - 30%-wt. penetration enhancers.

3. The TTS according to claim 2, **characterized in that** the penetration enhancer is a carboxylic acid.

4. The TTS according to claim 1, **characterized in that** the acrylate-based polymer material is a polymerisation product of acrylic acid and the esters thereof or of methacrylic acid and the esters thereof.

5. The TTS according to claim 4, **characterized in that** the esters of acrylic acid comprise as alcohol components straight-chain or branched alcohols having 4 - 10 carbons.

6. TTS according to claim 4, **characterized in that** the esters of acrylic acid comprise as alcohol components alcohols having 2 - 4 carbons.

7. The TTS according to claim 4, **characterized in that** the esters of methacrylic acid have amino alcohols as alcohol component.

8. TTS according to claim 4, **characterized in that** the acrylate-based polymer material is an optionally self-cross-linking acrylate-based copolymer.

9. TTS according to claim 1, **characterized in that** the system is provided with a removable protective layer.

## Revendications

1. Système thérapeutique transdermique (TTS) pour l'administration de galanthamine sur la peau comprenant une couche dorsale imperméable au composant actif et une couche auto-adhésive faisant office de réservoir, **caractérisé en ce que** la couche faisant office de réservoir comprend une matière polymère à base d'acrylate à concurrence de 40 à 80 % en poids, un plastifiant choisi parmi le groupe comprenant des alcools, des esters et des composés (poly)éthoxylés à concurrence de 0,1 à 30 % en poids et une base de galanthamine ou un de ses sels pharmaceutiquement acceptables à concurrence de 0,1 à 30 % en poids.

2. TTS selon la revendication 1, **caractérisé en ce que** la couche auto-adhésive faisant office de réservoir contient en outre un amplificateur de la pénétration à concurrence de 0,1 à 30 % en poids.

3. TTS selon la revendication 2, **caractérisé en ce que** l'amplificateur de la pénétration est un acide carboxylique.

4. TTS selon la revendication 1, **caractérisé en ce que** la matière polymère à base d'acrylate est un produit de polymérisation d'acide acrylique et de ses esters ou d'acide méthacrylique et de ses esters.

5. TTS selon la revendication 4, **caractérisé en ce que** les esters de l'acide acrylique comprennent, à titre de constituants alcooliques, des alcools à chaîne droite ou ramifiée contenant de 4 à 10 atomes de carbone.

6. TTS selon la revendication 4, **caractérisé en ce que** les esters de l'acide acrylique comprennent, à titre de constituants alcooliques, des alcools contenant de 2 à 4 atomes de carbone.

7. TTS selon la revendication 4, **caractérisé en ce que** les esters de l'acide méthacrylique présentent, à titre de fraction alcoolique, des aminoalcools.

8. TTS selon la revendication 4, **caractérisé en ce que** la matière polymère à base d'acrylate est un copolymère à base d'acrylate qui manifeste le cas échéant un pouvoir d'auto-réticulation.

9. TTS selon la revendication 1, **caractérisé en ce que** le système est muni d'une couche de protection amovible.
